# EUROPEAN PATENT APPLICATION

(11) **EP 0 992 245 A1**
(43) Date of publication of application: **12.04.2000**
(21) Application number: 98117598.7
(22) Date of filing: 16.09.1998
(51) Int. Cl.: A61K 49/04

(54) **Radio-contrast agents**

(71) Applicant: Goldham Bioglan Pharma GmbH, 86441 Zusmarshausen (DE)
(72) Inventor: Frank, Artur, 86441 Zusmarhausen (DE); Giannettino, Andreina, 00144 Roma (IT)
(74) Representative: Wibbelmann, Jobst, Dr., Dipl.-Chem.

(57) **Abstract**

An imaging or contrast agent comprising a stereoisomer of a compound with at least one chiral centre, wherein said stereoisomer is in stereoisomeric excess and causes fewer adverse side effects on administration, or is less chemotoxic, than at least one other stereoisomer of said chiral compound.

## Description

The present invention relates to radio-contrast agents and, in particular, to water soluble, non-ionic x-ray contrast agents or media.

Most known contrast or imaging agents are to some extent pharmacologically active and their use, thus, can cause adverse side effects in patients. These adverse pharmacological effects are often referred to as "chemotoxic" effects. It has been suggested that an agent's chemotoxic effect stems from its capacity to bind protein and its resulting ability to inhibit certain enzyme systems and to interfere with normal metabolic pathways by binding cell surface receptor proteins. For example, certain contrast agents have been shown to cause tachycardia and others to interfere with normal hemostatic processes.

Many non-ionic contrast agents include at least one chiral carbon atom and it is well known that the stereoisomers of optically active compounds can exhibit markedly different pharmacological properties. In particular, it is common for one stereoisomer of a pair to be markedly more active or chemotoxic than the other. For example, the L-form of the beta-adrenergic blocking agent popranolol, is a hundred times more potent than the D-enantiomer. It has also been suggested that the D-enantiomer of thalidomide is both safe and an effective sedative, even when used for the control of morning sickness during pregnancy, while the corresponding L-enantiomer is now believed to have been responsible for the racemic drug's notorious teratogenic effect.

The present invention is based upon the understanding that imaging or contrasting agents, ideally, should be pharmacologically inert and stems from a realisation that the above discussed properties of optically active compounds can be exploited in order to bring this ideal closer to realisation.

Accordingly, the present invention provides an imaging or contrast agent comprising a stereoisomer of a compound with at least one chiral centre, wherein said stereoisomer is in stereoisomeric excess and causes fewer adverse side effects on administration, or is less chemotoxic, than at least one other stereoisomer of said chiral compound. A chiral compound is understood to include a stereoisomeric excess of a particular stereoisomer when the latter is present in a greater proportion that it would be in the racemic compound.

Preferably, the compound includes a stereoisomeric excess of a single stereoisomer which causes fewer side effects or is less chemotoxic than at least one other stereoisomer of the compound. More preferably, said single stereoisomer causes the fewest adverse side effects, or is the least chemotoxic, of said chiral compound's stereoisomers.

In preferred embodiments, the stereoisomer in stereoisomeric excess has a lower affinity for a human receptor than at least one other stereoisomer of said chiral compound. Preferably, said single stereoisomer has the lowest affinity for a human receptor of said chiral compound's stereoisomers.

An advantage of contrast agents in accordance with the present invention is that they can be employed in greater quantities or concentrations than their equivalents which are either racemic, or consist of a more chemotoxic stereoisomer.

Said chiral compound need not be optically pure but, in preferred embodiments, greater than 50% thereof is in the form of said single stereoisomer. Preferably, greater than 70, 75, 80, 85, 90, 95, or 98% of said compound is in the form of said single stereo isomer. In compounds which have a single chiral carbon, said single stereoisomer is preferably in the R form.

The preferred family of compounds are derivatives of 2, 4, 6 - triiodoisophthalic acid. The preferred such compounds are 2,4,6-triiodo-1,3-benzenedicarboxamide derivatives, each with an R configured carbon located in an amido group bound to the five position in the benzene ring. The amido nitrogen is preferably directly bond to the ring.

Preferred examples of compounds which can be employed to form the imaging or contrast agents of the invention include iopamidol, iohexol, ioversol, iodixanol, iomeprol, iopentol, iopromid, itrolan and Gd-DTPA. The most preferred compound is iopamidol and the stereoisomer of this agent which must be in stereoisomeric excess is R-iopamidol. Currently, iopamidol is only available in its more chemotoxic S-stereoisomeric form.

### Example 1

### Preparation of R iopamidol [R-5-(α-hydroxypropionylamino)-2,4,6-triiodoisophthalic add di-(1,3 dihydroxyisopropylamide)].

400g (0.72 Mole) 5-amino-2,4,6-triiodoisophthalic acid should be added to 200 ml thionyl chloride, the mixture boiled and stirred for six hours, and the resulting solution then evaporated. The residue should then be dissolved in anhydrous ethyl acetate, and the solution should again be evaporated to dryness. The solid material should then be dissolved in 4000 ml ethyl acetate, and the solution stirred into an ice-cold solution of 500 g sodium chloride and 200 g sodium bicarbonate in 2.5 liters water. The organic phase should be separated from the aqueous solution, washed with aqueous sodium chloride solution, dried by contact with anhydrous calcium chloride, and evaporated to dryness.

The resulting 5-amino-2,4,6-triiodoisophthalyl chloride has a melting point of about 300°C when recrystallized from toluene.

300 g (0.503 mole) 5-amino-2,4,6-triiodoisophthalyl chloride should be dissolved in 1200 ml dimethylacetamide, and 187 g (1.26 mole) R-2 acetoxypropionyl chloride added dropwise to the solution at 3-5° C with agitation. The mixture should be permitted to stand overnight at ambient temperature and then should be evaporated in a vacuum to approximately 400 ml. The oily residue should be stirred into ice water to precipitate crystalline R-5-(α-acetoxypropionylamino) 2,4,6-triiodoisophthalyl chloride which should then be purified by suspension in warm alcohol free chloroform.

28.4 g (0.04 mole) of the purified R-5-(α-acetoxypropionylamino) 2,4,6-triiodoisophthalyl chloride should then be dissolved in 150 ml dimethylacetamide and 15 g (0.08 mole) tributylamine. The mixture should be heated to 50° and 9.1 g (0.01 mole) 1,3-dihydroxyisopropylamine (2-amino-1,3-propanediol) dissolved in 80 ml dimethylacetamide added drop by drop. The reaction should go completion within a few hours, and the reaction mixture should then be evaporated to dryness in a vacuum. The oily residue should be added to 350 ml methylene chloride with vigorous agitation, and the resulting precipitate filtered off and purified by repeated suspension in warm methylene chloride.

The resulting R-5-(α-acetoxypropionylamino)-2,4,6-triiodoisophthalic acid di-(1-3-dihydroxyisopropylamide) should then be dissolved in water and the solution decolourized with active carbon, adjusted to pH 11 with concentrated sodium hydroxide solution, heated to 40°C, and mixed with additional NaOH solution until the pH has stabilized, indicating the complete saponification of the acetoxy groups.

Sequential contact with cation and anion exchange resins will remove the salts from the saponification mixture, and the deionized solution should then be evaporated to dryness. The residue should be further purified by recrystallisation from ethanol to provide the required R-5-(α-hydroxxypropionylamino)-2,4,6-triiodoisophthalic acid di-(1-3-dihydroxyisopropylamide).

### Example 2

### Formulation for injection.

The ingredients set out in the following table were mixed together to provide a contrast agent composition in accordance with the present invention.
R-iopamidol (greater than 70% stereoisomeric purity, preferably greater than 98% stereoisomeric purity).
Ca-EDTA
Tromethamine.
HCl
Water for injection.

## Claims

1. An imaging or contrast agent comprising a stereoisomer of a compound with at least one chiral centre, wherein said stereoisomer is in stereoisomeric excess and causes fewer adverse side effects on administration, or is less chemotoxic, than at least one other stereoisomer of said chiral compound.

2. An imaging or contrast agent as claimed in claim 1, comprising a stereoisomeric excess of a single stereoisomer, wherein said single stereoisomer causes fewer adverse side effects, or is less chemotoxic, than at least one other stereoisomer of the chiral compound.

3. An imaging or contrast agent as claimed in claim 2, wherein said single stereoisomer causes the fewest adverse side effects, or is the least chemotoxic, of said chiral compound's stereoisomers.

4. An imaging or contrast agent as claimed in any of the preceding claims, wherein the stereoisomer in stereoisomeric excess has a lower affinity for a human receptor than at least one other stereoisomer of said chiral compound.

5. An imaging or contrast agent as claimed in claim 4, wherein said stereoisomer in stereoisomeric excess has the lowest affinity for a human receptor of the chiral compound's stereoisomers.

6. An imaging or contrast agent as claimed in any of the preceding claims comprising greater than 50% of a single stereoisomer.

7. An imaging or contrasting agent as claimed in claim 6, wherein greater than 70, 75, 80, 85, 90, 95 or 98% of said compound is in the form of a single stereoisomer.

8. An imaging or contrast agent as claimed in any of the preceding claims and comprising a derivitive of 2,4,6-triiodoisophthalic acid.

9. An imaging or contrast agent as claimed in claim 8, comprising a 2,4,6-triiodo-1-3-benzene dicarboxamide derivative with an R configured carbon located in an amido group bound to the 5 position in the benzene ring.

10. An imaging or contrast agent as claimed in claim 9, wherein the amido nitrogen is bound directly to a ring carbon atom.

11. An imaging or contrast agent as claimed in any of the preceding claims, wherein said compound has a single chiral carbon and said stereoisomer in stereoisomeric excess is in the R form.

12. An imaging or contrast agent as claimed in any of the preceding claims comprising iopamidol, iohexol, ioversol, iodixanol, iomeprol, iopentol, oipromid or itrolan.

13. An imaging or contrast agent as claimed in claim 12 comprising iopamidol.

14. An imaging or contrast agent comprising a stereoisomeric excess of R-iopamidol.
